# EUROPEAN PATENT APPLICATION

(11) **EP 2 386 270 A2**
(43) Date of publication of application: **16.11.2011**
(21) Application number: 11166179.9
(22) Date of filing: 16.05.2011
(51) Int. Cl.: A61F 2/00, A61F 2/04

(54) **System for diverticulitis treatment**

(30) Priority: 14.05.2010 US 780154
(71) Applicant: Tyco Healthcare Group, LP, Boulder, CO 80301 (US)
(72) Inventor: Alexander, Scott F., Westminster, CO 80020 (US); Johnson, Kristin D., Louisville, CO 80027 (US)
(74) Representative: HOFFMANN EITLE

(57) **Abstract**

A system for preventing recurrence of diverticulitis includes a semi-permeable sleeve configured to be placed within a colonic lumen relative to one or more diverticulum lining the colonic lumen. The system also includes at least first and second collars disposed on opposing ends of the semi-permeable sleeve. The collars are configured to be selectively expanded radially outward to anchor the semi-permeable sleeve within the colonic lumen. The semi-permeable sleeve is configured to prevent permeation of undesirable material from within the colonic lumen into the at least one diverticulum and allow permeation of desirable material from the exterior of the semi-permeable sleeve into the colonic lumen.

## Description

### BACKGROUND

### 1. Technical Field

The present disclosure relates generally to systems and methods for the treatment of diverticulitis. More particularly, the disclosure relates to a system and method for treating diverticulitis and preventing the recurrence of diverticulitis.

### 2. Background Of Related Art

Diverticulitis is a common gastrointestinal (GI) disease in the Western World. It is commonly believed that low fiber content in the Western diet causes the formation of diverticula - outpockets found primarily in the left colon, notably the sigmoid colon. These outpockets tend to occur where the vasa recta arteries penetrate the submucosal layer of the colon. About half of adults over the age of 60 have diverticula. Diverticulitis occurs when fecal matter becomes lodged in the diverticula pockets and causes infection. 10-25% of people with diverticula suffer an episode of diverticulitis.

Current treatment protocol for complicated diverticulitis is surgical resection. Currently, one third of all colostomies and/or colon resections are due to diverticulitis. Unfortunately, removing sections of the colon can impair the natural functioning of the colon leading to undesirable results such as diarrhea. Further, recovery periods following colon resection may be as long as several weeks and may incur significant expenses. The rationale for elective surgery is to prevent recurrent complicated diverticulitis and to reduce emergency procedures. However, 8.7% of patients receiving a colectomy due to diverticulitis will suffer a subsequent diverticulitis episode.

### SUMMARY

According to one embodiment of the present disclosure, a method of treating diverticulitis includes the steps of placing an endoscopic device within a colonic lumen relative to one or more diverticulum lining the colonic lumen and removing undesirable material from the one or more diverticulum utilizing the endoscopic device. The method also includes the steps of injecting a medicating agent into the one or more diverticulum utilizing the endoscopic device and sealing the one or more diverticulum from the colonic lumen.

According to another embodiment of the present disclosure, a method of treating diverticulitis includes the steps of placing an endoscopic device within a colonic lumen relative to one or more diverticulum and removing undesirable material from the one or more diverticulum utilizing the endoscopic device. The method also includes the steps of injecting a medicating agent into the one or more diverticulum utilizing the endoscopic device and placing a sleeve relative to the colonic lumen to seal the one or more diverticulum from the colonic lumen.

A method of treating diverticulitis includes the steps of placing an endoscopic device within a colonic lumen relative to one or more diverticulum and aspirating the one or more diverticulum utilizing an aspiration device in operable cooperation with the endoscopic device. The method also includes lavaging the one or more diverticulum utilizing a lavage device in operable cooperation with the endoscopic device and injecting a medicating agent into the one or more diverticulum utilizing the endoscopic device. The method also includes the step of placing a semi-permeable sleeve within the colonic lumen relative to the one ore more diverticulum. The semi-permeable sleeve is configured to prevent permeation of undesirable material from within the colonic lumen into the one or more diverticulum and to allow permeation of desirable material from the exterior of the semi-permeable sleeve into the colonic lumen.

According to another embodiment of the present disclosure, a system for preventing recurrence of diverticulitis includes a semi-permeable sleeve configured to be placed within a colonic lumen relative to one or more diverticulum lining the colonic lumen. The semi-permeable sleeve includes at least first and second collars disposed on opposing ends that are configured to be selectively expanded radially outward to anchor the semi-permeable sleeve within the colonic lumen. The semi-permeable sleeve is configured to prevent permeation of undesirable material from within the colonic lumen into the one or more diverticulum and to allow permeation of desirable material from the exterior of the semi-permeable sleeve into the colonic lumen.

According to another embodiment of the present disclosure, an apparatus for preventing recurrence of diverticulitis includes a sleeve configured to be disposed about the outside of the colon relative to one or more diverticulum lining the colonic lumen. The sleeve is placed laparoscopically and could include at least first and second collars disposed on opposing ends that are configured to selectively anchor the sleeve about the colonic lumen or the sleeve may be anchored by compression or a tacking means. The sleeve is configured to compress the one or more diverticulum toward the colonic lumen to prevent permeation of undesirable material from within the colonic lumen into the one or more compressed diverticulum.

According to another embodiment of the present disclosure, an apparatus for preventing recurrence of diverticulitis includes a semi-permeable sleeve configured to be placed within a colonic lumen relative to at least one diverticulum lining the colonic lumen. The semi-permeable sleeve includes a proximal collar and a distal collar disposed on opposing ends that are configured to be selectively expanded radially outward to anchor the semi-permeable sleeve proximally at least partially within the descending colon and distally at least partially within the rectum such that semi-permeable sleeve is disposed at partially along the length of the sigmoid colon. The semi-permeable sleeve is configured to prevent permeation of undesirable material from within the colonic lumen into the one or more diverticulum and to allow permeation of desirable material from the exterior of the semi-permeable sleeve into the colonic lumen.

With reference to all of the various embodiments described above, the sleeve may be held or anchored in place by any one or more of a compressive means, an expansive means, a tacking means (e.g., physical tacks), adherence via the use of energy to fuse the sleeve to the wall of the colonic lumen (e.g., descending colon, transverse colon, ascending colon, cecum colon), and/or adhesives. In some embodiments, the surface of the sleeve that is in contact with the colonic lumen may be modified with an injection or coating of a suitable agent to encourage tissue in-growth of the colonic lumen that operates to anchor the sleeve. In some embodiments, the surface of the sleeve that is in contact with the colonic lumen may be modified with a control coating to prohibit formation of biofilms on the sleeve surface.

### BRIEF DESCRIPTION OF THE DRAWINGS

Various embodiments of the present disclosure are described herein with reference to the drawings wherein:

Figs. 1A and 1B show a diagram of an endoscopic device for treating diverticulitis in accordance with various embodiments of the present disclosure;

Figs. 2A-2B show the endoscopic device of Fig. 1A in use to illustrate a method of treating diverticulitis in accordance with an embodiment of the present disclosure;

Figs. 3A and 3B show a perspective view of a sleeve configured to prevent the recurrence of diverticulitis according to various embodiments of the present disclosure;

Fig. 4 shows the sleeve of Fig. 3A placed within a colonic lumen;

Fig. 5 shows a sleeve disposed about a colonic lumen in accordance with another embodiment of the present disclosure;

Fig. 6 illustrates a method of preventing recurrence of diverticulitis utilizing a bipolar forceps according to an embodiment of the present disclosure;

Fig. 7 illustrates a method of preventing recurrence of diverticulitis utilizing elastic bands according to an embodiment of the present disclosure;

Figs. 8A and 8B illustrate a method of preventing recurrence of diverticulitis utilizing removal of diverticula according to an embodiment of the present disclosure; and

Fig. 9 illustrates a method of preventing recurrence of diverticulitis utilizing a filling agent in accordance with an embodiment of the present disclosure.

### DETAILED DESCRIPTION

Embodiments of the presently disclosed diverticulitis treatment devices are described in detail with reference to the drawings, in which like reference numerals designate identical or corresponding elements in each of the several views. As used herein the term "distal" refers to that portion of the devices, or component thereof, farther from the user while the term "proximal" refers to that portion of the devices or component thereof, closer to the user. In addition, the term "treatment" includes, but is not limited to, "prevention".

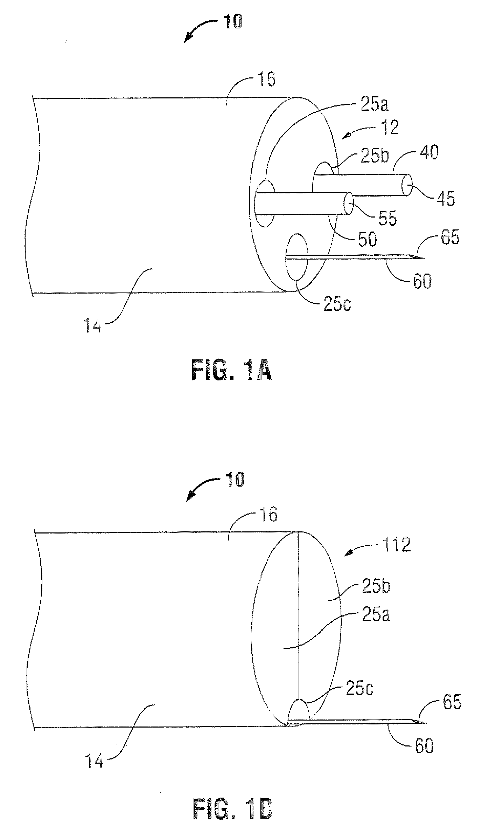

An endoscopic diverticulitis treatment device in accordance with embodiments of the present disclosure is referred to in the figures as reference numeral 10. Referring initially to Fig. 1, endoscopic treatment device 10 includes a shaft 14 having a handle portion at a proximal end (not shown) and a working portion 12 at a distal end 16. Shaft 14 may be semi-rigid or semi-flexible such that manipulation of device 10 within an anatomical structure (e.g., colon, sigmoid colon, descending colon, rectum, colonic lumen, etc.) may easily be facilitated.

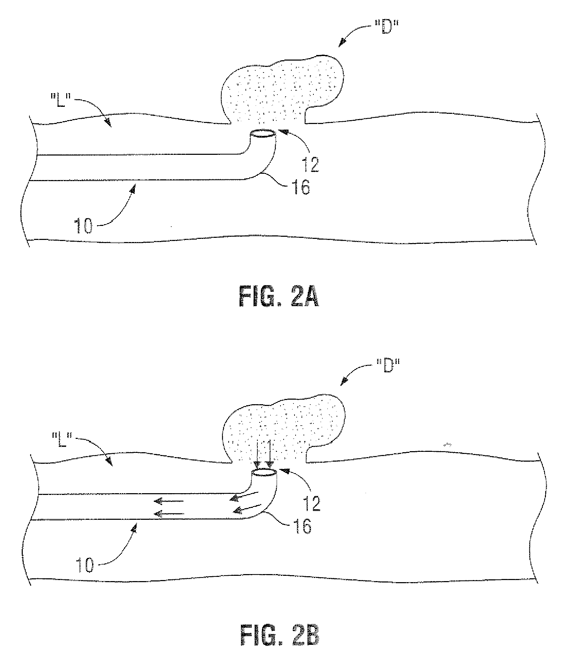

As shown in Fig. 1A, working portion 12 includes a plurality of lumens 25a, 25b, 25c disposed coaxially through shaft 14 to facilitate proximal and/or distal movement of any number of surgical devices 40, 50, 60 therethrough. In this manner, devices 40, 50, 60 may be moved distally through shaft 14 to extend distally from working portion 12 such that devices 40, 50, 60 may be utilized to treat a desired surgical site (e.g., diverticula "D" shown in Figs. 2A-2D), as discussed in further detail below.

As will be discussed in greater detail below, in some embodiments, device 40 may be a suction device and/or an aspirator configured to remove undesirable materials from diverticula for purposes of treating diverticulitis. For example, aspirator 40 may be used to remove, via suction and/or aspiration, feces from a diverticulum. With this purpose in mind, aspirator 40 may be operably connected to a suitable suction source or suction pump (not shown) to facilitate suction of material via a distal end 45 thereof. In some embodiments, device 50 may be an irrigation or lavage device configured to flush out the cavity of an organ or wound utilizing fluid (e.g., water or an antiseptic agent) expelled from a distal end 55. More specifically, lavage device 50 may be operably connected to a suitable fluid pump (not shown) to facilitate the expulsion of fluid from distal end 55 of lavage device 50 for purposes of flushing out undesirable material from a diverticulum, as discussed in further detail below. Device 60 may be a material delivery device such as, for example, a needle adapted to deliver a medicating agent (e.g., antibiotic) from a distal end 65 thereof. More specifically, material delivery device 60 may be utilized to deliver a medicating agent into a diverticulum for purposes of treating diverticulitis, as will be discussed in further detail below. Although not explicitly shown, device 10 may be configured such that any one of instruments 40, 50, 60 may be user-operated from a proximal end (not shown) of the device 10.

Fig. 1B shows device 10 of Fig. 1A having a working portion 112 according to an alternative embodiment of the present disclosure. In this embodiment, working portion 112 may be configured such that passages 25a and 25b serve to operate identical to aspirator 40 and lavage device 50, respectively. More particularly, in this embodiment, passage 25a may operate as an aspirator configured to remove undesirable materials from diverticula for purposes of treating diverticulitis and passage 25b may operate as a lavage device configured to expel fluid therefrom to flush out diverticula for purpose of treating diverticulitis. Passage 25c, as described above with respect to working portion 12, facilitates passage of device 60 through shaft 14.

Figs. 2A-2D illustrate a method of treating diverticulitis according to some embodiments of the present disclosure. For purposes of discussion, the method illustrated by Figs. 2A-2D is shown to include the use of device 10 of Fig. 1A and/or Fig. 1B including instruments 40, 50, 60. However, the method illustrated by Figs. 2A-2D and described in detail below, may be employed utilizing any suitable endoscopic device in conjunction with any suitable surgical instruments for effecting the treatment of diverticulitis detailed below.

Referring to Fig. 2A, device 10 initially is entered into a colonic lumen "L" (e.g., sigmoid colon, descending colon, rectum, etc.) and placed relative to a diverticulum "D" such that working portion 12 is proximate the target diverticulum "D". Once appropriately placed, device 10 is utilized to aspirate or suction the diverticulum "D", as depicted by Fig. 2B. In this scenario, aspirator 40 is placed proximate to or distal to working portion 12 of device 10 and utilized to remove undesirable materials (e.g., feces) from the diverticulum "D" through use of aspiration and/or suction generated by a suitable suction pump (not shown) operably connected to the aspirator 40.

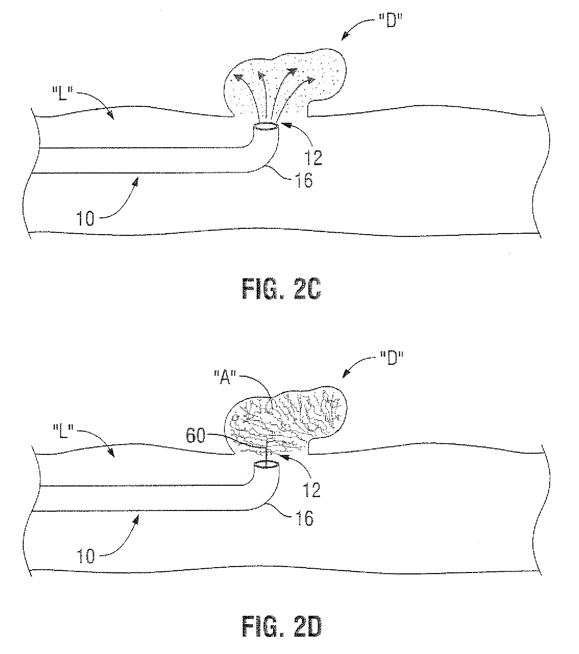

Subsequent to, prior to, or substantially simultaneously to aspirating a diverticulum "D", device 10 may be utilized to lavage or irrigate the diverticulum "D", as depicted by Fig. 2C. In this scenario, lavage device 50 is placed proximate to or distal to working portion 12 of device 10 and utilized to "wash out" or flush the diverticulum "D" of any undesirable materials (e.g., feces, blood, etc.) using the pressurized expulsion of fluid (e.g., water, saline, etc.) generated by a suitable fluid pump (not shown) operably connected to the lavage device 50. In some scenarios, the step of flushing a diverticulum "D" depicted in Fig. 2C may not be necessary, for example, depending on the result of the aspiration step depicted in Fig. 2B. Like-wise, in some scenarios, flushing a diverticulum "D" may be performed prior to the step of aspirating the diverticulum "D" and, depending on the result thereof, may render the aspirating step of Fig. 2B unnecessary.

Once the diverticula "D" has been aspirated and/or flushed, as depicted in Figs. 2B and 2C, respectively, device 10 may be utilized to treat the diverticulum "D" with a medicating agent "A", as depicted by Fig. 2D. In this scenario, treatment device 60 is placed proximate to or distal to working portion 12 of device 10 and utilized to inject a medicating agent such as an antibiotic into the diverticulum "D". The injection of medicating agent into the diverticulum "D" may be performed to hasten healing and/or to prevent infection of the diverticulum "D".

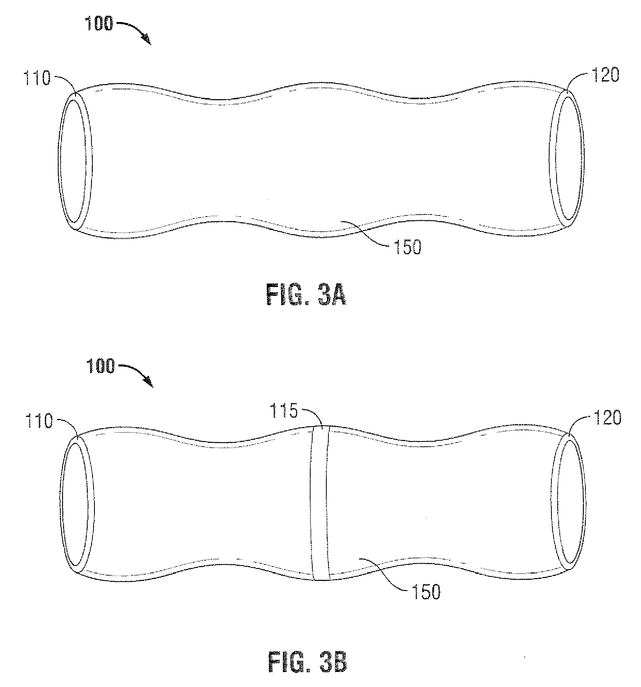

Once a diverticulum "D" has been aspirated and/or flushed and treated with medicating agent(s), as described hereinabove, various methods and/or devices may be employed to prevent recurrences of diverticulitis. These various methods and/or devices are described below with reference to Figs. 3-9 and may, in some embodiments, be performed subsequent to the treatment method described above with respect to Figs. 2A - 2D.

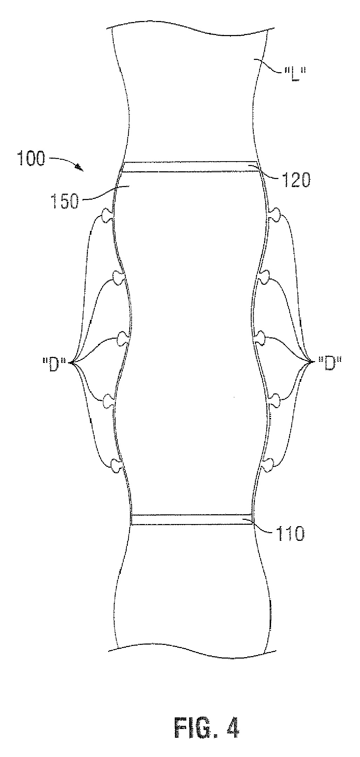

Referring now to Fig. 3A, a diverticulitis treatment device is referenced generally as 100 and is configured to be placed within and at least partially along the length of a colonic lumen "L", such as the sigmoid colon. As discussed in further detail below, device 100 is configured to prevent undesirable material (e.g., feces) passing through the colonic lumen "L" from entering or getting lodged within diverticula lining the colonic lumen "L" while leaving the passage or movement of such undesirable material through the colonic lumen "L" uninterrupted. With this purpose in mind, device 100 includes a proximal collar 110 and a distal collar 120 disposed at opposing ends of a flexible semi-permeable sleeve 150. Collars 110, 120 are selectively expandable radially outward via any suitable method to anchor sleeve 150 within the colonic lumen "L" (see Fig. 4). In some embodiments, sleeve 150 may be anchored proximally (e.g., via collar 110) at least partially in the descending colon, transverse colon, ascending colon, or cecum colon. In some embodiments, sleeve 150 may be anchored distally (e.g., via collar 120) at least partially in the rectum.

With reference to Fig. 3B, device 100 may include at least one collar, referenced as 115, disposed between collars 110 and 120 for added anchoring strength of semi-permeable sleeve 150 relative to colonic lumen "L". Although not shown, it is in the spirit of this disclosure that any number of collars may be incorporated between proximal and distal collars 110 and 120, respectively, to further aid in anchoring sleeve 150 relative to colonic lumen "L".

In use, a suitable endoscopic device (e.g., device 10, an endoscope, etc.) is used to place sleeve 150 within the interior of colonic lumen "L". For example, the sleeve 150 may be disposed on a suitable endoscopic placement device (not shown) configured to be moved proximally and/or distally within the colonic lumen "L". Once the placement device is properly positioned within the colonic lumen "L", collars 110, 120 may be selectively expanded radially outward to at least partially bias the interior walls of lumen "L", thereby anchoring sleeve 150 within the colonic lumen "L" at least partially along a length thereof. Upon anchoring of sleeve 150 within lumen "L", the placement device is retracted from the colonic lumen "L" while the sleeve 150 remains anchored. In this manner, the sleeve 150 is disposed along at least the portion of the colonic lumen "L" having diverticula "D" infected with diverticulitis, as depicted in Fig. 4.

Sleeve 150 is configured to substantially line the interior walls of the colonic lumen "L" as shown in Fig. 4 such that the diverticulitis "D" is sealed from undesirable material (e.g., feces) within the colonic lumen "L" while leaving passage of such undesired material through the sigmoid colon (including the portion lined by the sleeve 150) uninterrupted. More specifically, to prevent the recurrence of diverticulitis, sleeve 150 is formed of a semi-permeable material (e.g., fabric, woven mesh, etc.) that allows inward permeation from the exterior of the sleeve 150 into the colonic lumen "L" while preventing outward permeation from the interior of the colonic lumen "L" through sleeve 150. In particular, this configuration ensures that permeation of feces into the diverticula "D" lining the colonic lumen "L" is prevented while permeation of colon-produced mucous, vital to the health of the colon, is allowed. One example suitable material that can be made to meet these functional requirements is polytetrafluoroethylene (PTFE), particularly a woven form of PTFE. Particularly envisaged as being a suitable material is expanded PTFE (ePTFE), preferably in woven form.

Alternatively, sleeve 150 may be configured such that both inward and outward permeation therethrough is prevented. In some embodiments, the placement and subsequent anchoring of sleeve 150 within the colonic lumen "L" is performed subsequent to the treatment method described above with reference to Figs. 2A-2D. That is, once the target diverticula "D" are aspirated and/or flushed and treated with a medicating agent (e.g., antibiotic), the sleeve 150 is anchored within the colonic lumen "L" as described above with respect to Fig. 4 to prevent the recurrence of diverticulitis episodes. Sleeve 150 may be formed of or coated with an antibiotic agent such as, for example, a silver-based weave and/or sleeve 150 may be coated with an antimicrobial. Also, sleeve 150 may be treated and/or coated with a suitable agent configured to prevent tissue in-growth through sleeve 150 that may cause sleeve 150 to become clogged, thereby preventing desired permeation therethrough.

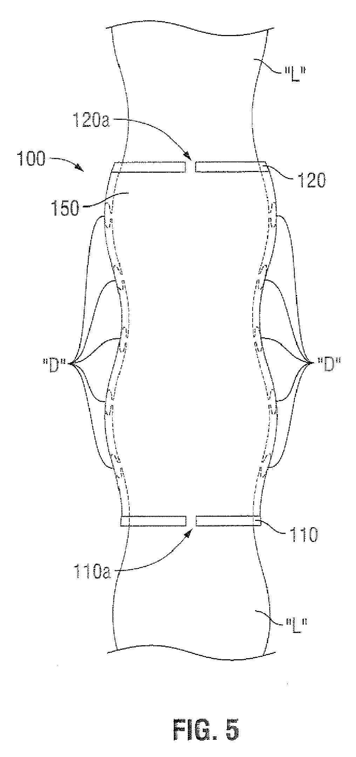

Referring now to Fig. 5, another embodiment of device 100 is shown and is configured to be placed about the exterior of the colonic lumen "L" to compress diverticula "D" toward the colonic lumen "L" such that the cavity or outpocket of each diverticulum "D" is collapsed and, thus, is unable to hold any contents. In this manner, undesirable material (e.g., feces) is unable to become lodged within diverticulum "D" compressed by sleeve 150. In the illustrated embodiment, collars 110 and 120 include open portions 110a and 120a, respectively, such that collars 110 and 120 are generally c-shaped to allow for collars 110, 120 to be easily placed about the exterior of the colonic lumen "L" (rather than having to be slid over the colonic lumen "L"), as depicted in Fig. 5. Once appropriately placed, collars 110, 120 may be compressed or clamped about the colonic lumen "L" to anchor sleeve 150 thereto. In the anchored configuration, sleeve 150 is sufficiently tight about the exterior of the colonic lumen "L" such that any diverticula disposed between the sleeve 150 and the colonic lumen "L" are compressed as described above. In order to ease sleeve placement, the sleeve 150 may be formed into a general c-shape, preferably that wraps most of the way, but not all of the way, about the colon or it can be in the form of a sheet wrapped about the colon and held in place by the collars 110, 120. In this scenario, sleeve 150 may be but is not necessarily semi-permeable since it is not placed within the colonic lumen "L". In some embodiments, the compression of diverticula "D" is performed subsequent to the treatment method described above with reference to Figs. 2A-2D. That is, once the diverticula "D" are aspirated and/or flushed and treated with a medicating agent (e.g., antibiotic), the diverticula "D" are compressed by sleeve 150 as described above with respect to Fig. 5 to prevent undesirable material from entering the diverticula "D", thereby preventing the recurrence of diverticulitis episodes. Other means may be utilized in addition to or in place of collars 110, 120 for purposes of anchoring sleeve 150 to colonic lumen "L". These means could include adhesives, a tacking means, and/or a surface modified to encourage adhesion between the colonic lumen "L" and sleeve 150.

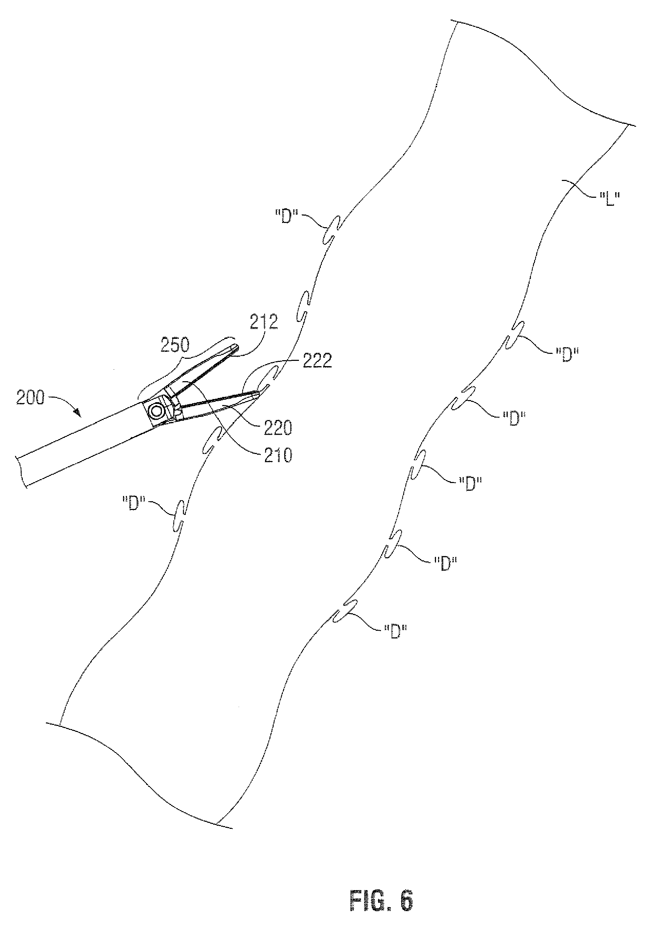

Referring now to Fig. 6, an electrosurgical device 200 is shown and may be employed to prevent the recurrence of diverticulitis "D". In the illustrated embodiment, electrosurgical device 200 includes an end effector assembly 250 having opposing jaw members 210 and 220 adapted to grasp tissue therebetween. Jaw members 210 and 220 include electrically conductive tissue sealing surfaces 212 and 222, respectively, operably connected to an electrosurgical energy source (not shown) and adapted to apply electrosurgical energy supplied from the energy source through tissue disposed between jaw members 210, 220 in a bipolar manner. One such source of electrosurgical energy is described in commonly-owned U.S. Patent No. 6,033,399 entitled "ELECTROSURGICAL GENERATOR WITH ADAPTIVE POWER CONTROL". In use, the end effector assembly 250 is used to grasp one or more diverticulum "D" and apply electrosurgical energy therethrough to effect a tissue seal. That is, each diverticulum "D" treated by the end effector assembly 250 is sealed (e.g., fluidly sealed) from the interior of the colonic lumen "L" such that undesirable material from the colonic lumen "L" (e.g., feces) is prevented from lodging into the diverticulum "D". In some embodiments, the sealing of diverticula "D" is performed subsequent to the treatment method described above with reference to Figs. 2A-2D. That is, once the diverticula "D" are aspirated and/or flushed and treated with a medicating agent (e.g., antibiotic), the diverticula "D" are sealed as described above with respect to Fig. 6 to prevent undesirable material from entering the diverticula "D", thereby preventing the recurrence of diverticulitis episodes.

For a more detailed description of a bipolar forceps including opposing jaw members that cooperate to grasp tissue therebetween, such as electrosurgical device 200, reference is made to commonly-owned Patent Publication No. 2003/0229344, filed on February 20, 2003, entitled VESSEL SEALER AND DIVIDER AND METHOD OF MANUFACTURING THE SAME.

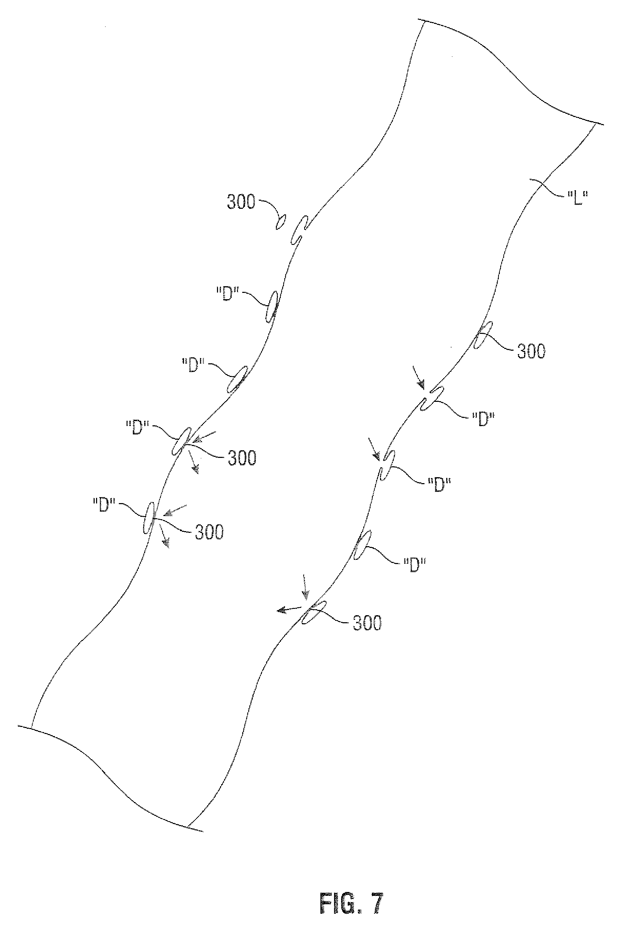

Referring now to Fig. 7, a method of preventing the recurrence of diverticulitis according to one embodiment includes the use of elastic bands 300 to seal diverticula "D" from the colonic lumen "L". More specifically, elastic bands 300 may be wrapped around the base of a diverticulum "D" to constrict the outpocket, thereby sealing the diverticulum "D" from the colonic lumen "L". In this manner, undesirable material (e.g., feces) is prevented from entering the diverticulum "D" from the colonic lumen "L". Elastic band 300 may be, for example, any suitable elastic material (e.g., a rubber band or the like). For purposes of contrast, Fig. 7 illustrates select diverticula "D" including an elastic band 300 wrapped therearound as well as select diverticula "D" lacking an elastic band 300 wrapped therearound. As depicted by directional arrows in Fig. 7, diverticula "D" treated with an elastic band 300 are sealed from colonic lumen "L" such that undesirable material is prevented from entering such diverticula "D". In contrast, diverticula "D" not treated with an elastic band 300 are not sealed from the colonic lumen "L" and, as such, undesirable material is not prevented from entering such diverticula "D" as depicted by directional arrows in Fig. 7. In some embodiments, the banding of diverticula "D" with elastic bands 300 is performed subsequent to the treatment method described above with reference to Figs. 2A-2D. That is, once the diverticula "D" are aspirated and/or flushed and treated with a medicating agent (e.g., antibiotic), the band 300 is wrapped around the base of each diverticulum "D" as described above with respect to Fig. 7 to prevent undesirable material from entering each diverticulum "D", thereby preventing the recurrence of diverticulitis episodes.

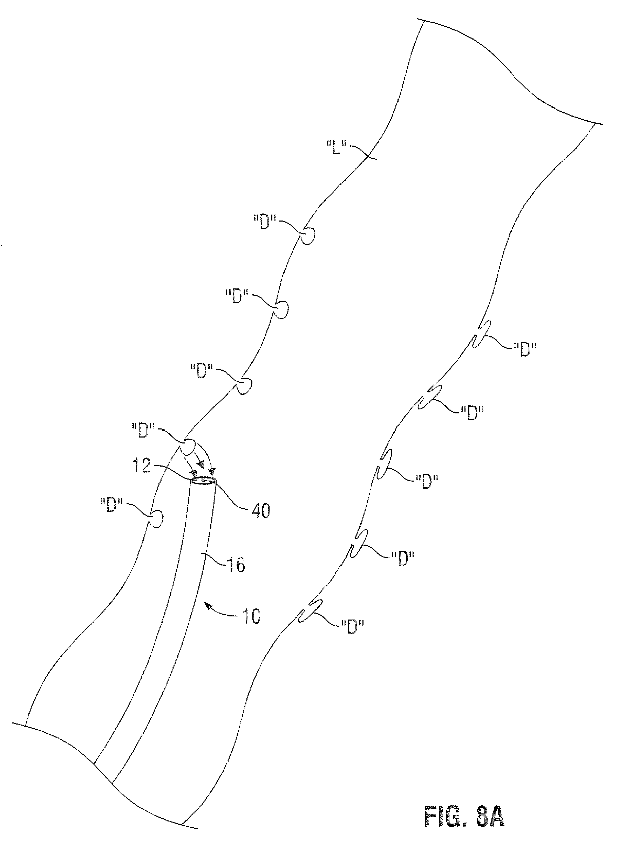

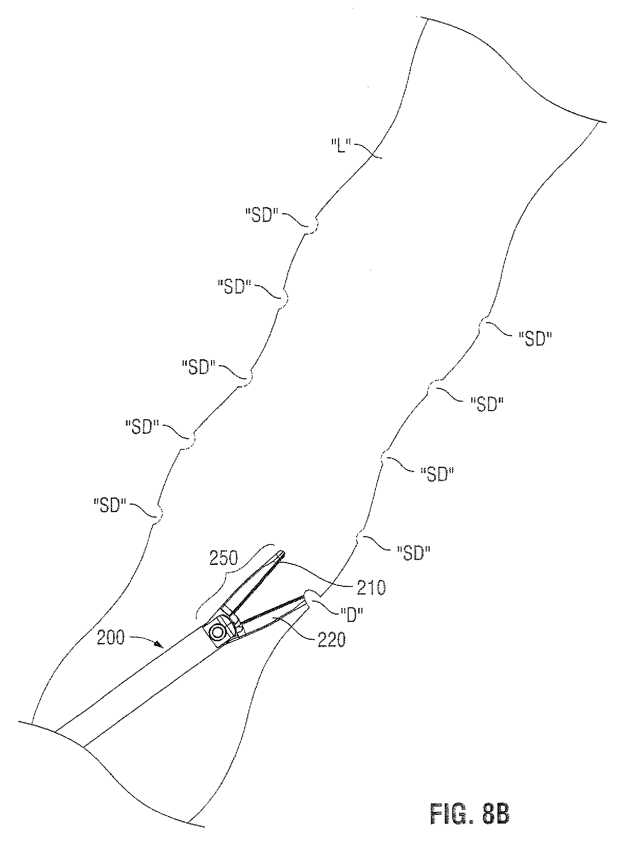

Referring now to Figs. 8A and 8B, a method of preventing the recurrence of diverticulitis according to one embodiment includes removal of diverticula "D" internally within the colonic lumen "L". More specifically, a suction device (e.g., aspirator 40) is utilized to pull each diverticulum "D" into the colonic lumen "L", as depicted in Fig. 8A, and a suitable jaw-type instrument or forceps (e.g., electrosurgical device 200) is utilized to sever the pulled-in diverticula "D" from the colonic lumen "L". In this scenario, device 200 may include a tissue cutting mechanism such as, for example, a selectively advanceable knife (not shown) and/or an electrical cutting device (not shown).

In use, as illustrated in Fig. 8A, device 10 may be placed within the colonic lumen "L" such that the working portion 12 is proximate the desired diverticula "D" so that aspirator 40 may be utilized to suction each diverticulum "D" into the interior of colonic lumen "L" such that each diverticulum "D" is disposed in an "inside-out" type configuration. Once pulled into the colonic lumen "L", as best shown in Fig. 8A, device 200 may be used to grasp each pulled-in diverticulum "D" between jaw members 210, 220 and subsequently sever each grasped diverticulum (referenced in Fig. 8B as "SD") utilizing the tissue cutting mechanism, as depicted in Fig. 8B.

For a more detailed description of a forceps including a selectively advanceable tissue cutting mechanism, such as device 200, reference is made to commonly-owned Patent Publication No. 2003/0229344, filed on February 20, 2003, entitled VESSEL SEALER AND DIVIDER AND METHOD OF MANUFACTURING THE SAME. In some embodiments, the pulled-in diverticula "D" may be severed from the colonic lumen "L" using a conventional cutting mechanism (not shown) such as, for example, a surgical knife. In some embodiments, the above described pulling in and subsequent severing of diverticula "SD" is performed subsequent to the treatment method described above with reference to Figs. 2A-2D. That is, once each diverticulum "D" is aspirated and/or flushed and treated with a medicating agent (e.g., antibiotic), each diverticulum "D" is suctioned or pulled into the colonic lumen "L" and subsequently severed utilizing device 200 or a suitable cutting mechanism as described above with respect to Figs. 8A and 8B. In this way, undesirable material is prevented from entering the diverticula "D" from the colonic lumen "L", thereby preventing the recurrence of diverticulitis episodes.

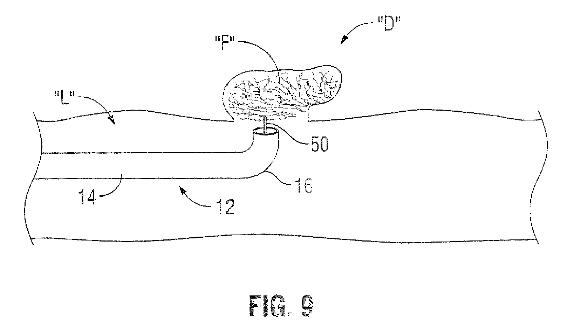

Referring now to Fig. 9, a method of preventing the recurrence of diverticulitis according to another embodiment includes injecting or placing a plug or filler agent "F" into each diverticulum "D" that is configured to occupy the diverticulum "D" and subsequently solidify therein. In this manner, undesirable material is prevented from entering the already-occupied diverticulum "D". Agents configured for this use may include, but are not limited to, non-adhesive embolic agents, cements, etc. The filler agent may be delivered to the target diverticula utilizing, for example, any one of the aspirator 40, the lavage device 50 (as depicted in Fig. 9), or the needle 60 as described above with respect to Figs. 2A-2D.

The described embodiments of the present disclosure are intended to be illustrative rather than restrictive, and are not intended to represent every embodiment of the present disclosure. Various modifications and variations can be made without departing from the spirit or scope of the disclosure as set forth in the following claims both literally and in equivalents recognized in law.

## Claims

1. A system for preventing recurrence of diverticulitis, comprising:
a semi-permeable sleeve configured to be placed within a colonic lumen relative to at least one diverticulum lining the colonic lumen;
at least first and second collars disposed on opposing ends of the semi-permeable sleeve and configured to be selectively expanded radially outward to anchor the semi-permeable sleeve within the colonic lumen,
wherein the semi-permeable sleeve is configured to prevent permeation of undesirable material from within the colonic lumen into the at least one diverticulum and allow permeation of desirable material from the exterior of the semi-permeable sleeve into the colonic lumen.

2. A system according to claim 1, wherein the semi-permeable sleeve includes at least a third collar disposed between the at least first and second collars.

3. A system according to claim 1 or 2, wherein the semi-permeable sleeve is configured to be placed at least partially along the length of the sigmoid colon.

4. A system according to claim 1, 2 or 3, wherein the semi-permeable sleeve is configured to be anchored proximally at least partially in the descending colon.

5. A system according to claim 1, 2, 3 or 4, wherein the semi-permeable sleeve is configured to be anchored distally at least partially in the rectum.

6. A system according to any one of the preceding claims, wherein the semi-permeable sleeve is configured to be anchored between the descending colon and the rectum.

7. A system according to any one of the preceding claims, wherein the semi-permeable sleeve is configured to be inserted into the colonic lumen transanally via an endoscopic device adapted to be selectively advanced within and retracted from the colonic lumen.

8. A system according to any one of the preceding claims, wherein the semi-permeable sleeve includes a weave of silver-based material disposed thereon.

9. A system according to any one of the preceding claims, wherein the semi-permeable sleeve is coated with an antimicrobial to prevent infection of the colonic lumen.

10. A system according to any one of the preceding claims, wherein the semi-permeable sleeve is coated with an agent configured to prevent in-growth of tissue from the colonic lumen.

11. A system according to any one of the preceding claims, wherein the undesirable material is feces.

12. A system according to any one of the preceding claims, wherein the desirable material is mucous produced by the colon.

13. A system according to any one of the preceding claims, wherein the semi-permeable sleeve is comprised of at least one of a fabric and a woven mesh.

14. A system according to any one of the preceding claims, wherein the first and second collars are configured to be selectively expanded radially outward to anchor the semi-permeable sleeve proximally at least partially within the descending colon and distally at least partially within the rectum such that the semi-permeable sleeve is disposed at least partially along the length of the sigmoid colon.

15. An apparatus for preventing recurrence of diverticulitis, comprising:
a sleeve configured to be disposed at least mostly about a colonic lumen relative to at least one diverticulum lining the colonic lumen;
at least first and second collars disposed on opposing ends of the sleeve and configured to selectively anchor the sleeve about the colonic lumen,
wherein the sleeve is configured to compress the at least one diverticulum toward the colonic lumen to prevent permeation of undesirable material from within the colonic lumen into the at least one compressed diverticulum.

16. An apparatus according to claim 15, wherein the first and second collars are generally c-shaped and configured to be clamped about the colonic lumen.

17. An apparatus according to claim 15 or 16, wherein the sleeve includes at least a third collar disposed between the at least first and second collars.
